# EUROPEAN PATENT APPLICATION

(11) **EP 4 464 371 A1**
(43) Date of publication of application: **20.11.2024**
(21) Application number: 23737194.3
(22) Date of filing: 09.01.2023
(51) Int. Cl.: A61N 5/06

(54) **DEVICE AND METHOD FOR REGULATING RELEASE OF ADENOSINE IN ORGANISM, AND USE**

(30) Priority: 10.01.2022 CN 202210021891
(71) Applicant: Wenzhou Ventura Technology Co., Ltd., Wenzhou, Zhejiang 325088 (CN)
(72) Inventor: CHEN, Jiangfan, Wenzhou, Zhejiang 325027 (CN); XU, Tao, Wenzhou, Zhejiang 325027 (CN); ZHOU, Xuzhao, Wenzhou, Zhejiang 325027 (CN); HE, Yan, Wenzhou, Zhejiang 325027 (CN); GUO, Wei, Wenzhou, Zhejiang 325027 (CN); CAI, Xiaohong, Wenzhou, Zhejiang 325027 (CN); YAO, Zhimo, Wenzhou, Zhejiang 325027 (CN); LI, Zhihui, Wenzhou, Zhejiang 325027 (CN); ZHANG, Yi, Wenzhou, Zhejiang 325035 (CN); LIU, Yuntao, Wenzhou, Zhejiang 325035 (CN); YU, Chenyi, Wenzhou, Zhejiang 325027 (CN); QU, Jia, Wenzhou, Zhejiang 325027 (CN)
(74) Representative: Elzaburu S.L.P.
(86) International application number: PCT/CN2023/071395
(87) International publication number: WO 2023/131332

(57) **Abstract**

A device and method for regulating the release of adenosine in an organism, and the use thereof. The device comprises a stimulus generator for generating stimuli of a specific frequency. The use of the device of the present invention in an organism can effectively increase the concentration of adenosine in the brain of the organism, thereby providing an effective strategy for the improvement of sleep disorders.

## Description

### Field of the invention

The present invention relates to a field of adenosine regulation, in particular to the device and method for regulating the release of adenosine in an organism, and the use thereof.

### Background of the invention

Adenosine and derivatives thereof are widely found in nature and play an indispensable role in the regulation of various biological behaviors. As early as 1997, McCharty's team firstly reported in *Science* that the concentration of extracellular adenosine varies with different body states of sleep-wake, and the accumulation of adenosine concentration will increase the pressure for sleep, which will lead to sleep. However, up to now the development of adenosine anti-insomnia drugs has not made substantial progress due to the wide expression of the adenosine receptors in systemic tissues and organs, high side effects, poor specificity, desensitization/tolerance of the agonists, etc.

According to the survey report of the Chinese Sleep Research Society in 2021, currently about 40% of the total population of our country suffers from varying degrees of sleep disorder. Chronic and severe insomnia will greatly damage the work efficiency and physical and mental health of patients. The losses of various accidents and incidents caused by sleep disorder amount to hundreds of billions of RMB yuan every year. Long-term insomnia is also an important inducing factor for a variety of neuropsychiatric diseases. With the rapid development of the economy of our country, the occurrence of sleep disorder has shown a generalized and younger trend.

At present, the main means of clinical treatment for insomnia is drug treatment, but there are some key problems such as drug persistence, drug dependence, peripheral side effects, etc. In addition to drug treatment, insomnia can also be treated with cognitive behavioral therapy and physical therapy of bright light therapy. The effect of cognitive behavioral therapy is limited and slow to take effect; while the effect of bright light therapy is weak.

### Summary of the invention

The technical problem to be solved in the present invention is that at present there is no safe and effective strategy for increasing the concentration of adenosine in an organism or precisely improving sleep disorder.

In order to solve the above-mentioned technical problem, the present inventor has found that light flicker stimulus can effectively increase the concentration of adenosine in the brain, thereby providing an effective strategy for improving sleep disorder. Specifically, the present invention provides the following technical solutions:
In one aspect, the present invention provides a device for regulating the release of adenosine in an organism, comprising a stimulus generator for generating a sound of 1-100 Hz and/or for generating a light flicker of 1-100 Hz and/or a full-screen visual stimulus.

The device described in the present invention, wherein the stimulus generator comprises visual stimulus (light flicker, etc.) generator and/or acoustic stimulus generator.

Optionally, the visual generator emits a visual stimulus of 20-80 Hz.

Optionally, the frequency of the visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz. Optionally, the frequency of the visual stimulus comprises 30-60 Hz.

Optionally, the frequency of the visual stimulus comprises one or more of 30 Hz, 35 Hz, 40 Hz, 45 Hz, 50 Hz, 55 Hz and 60 Hz.

Optionally, the frequency of the visual stimulus comprises 35-45 Hz.

Optionally, the frequency of the visual stimulus comprises one or more of 35 Hz, 40 Hz and 45 Hz.

Optionally, the frequency of the visual stimulus is 40 Hz.

Optionally, the white light illuminance of the visual stimulus is 2000 lux or more.

Optionally, the wavelength of the monochromatic light of the visual stimulus is 480-530 nm.

Optionally, the duty ratio of the visual stimulus is 20%-80%, preferably 30%-70%, or 40%-70%, or 45-55%.

Optionally, the duty ratio of the visual stimulus is selected from one or more of 20%, 25%, 30%, 35%, 40%, 45%, 50%, 55%, 60%, 65%, 70%, 75%, and 80%.

Optionally, the duty ratio of the visual stimulus is 50%.

The device described in the present invention, wherein the visual stimulus generator comprises a lamp and/or a display screen.

Optionally, the lamp comprises CCFL lamp, LED lamp and/or OLED lamp. Optionally, the display screen comprises CCFL display screen, LED display screen, and/or OLED display screen.

Optionally, the lamp emits white light and/or monochromatic light.

Optionally, the monochromatic light comprises one or more of ultraviolet light, blue light, green light, red light and near-infrared ray; preferably blue light and/or green light.

Optionally, the center wavelength of the ultraviolet light is 315-400 nm; e.g., 320-395 nm, or 325-390 nm, or 330-385 nm, preferably 380 nm.

Optionally, the center wavelength of the blue light is 450-490 nm; e.g., 455-488 nm, or 460-485 nm, preferably 480 nm;

Optionally, the center wavelength of the green light is 500 nm~560 nm; e.g., 505 nm~555 nm, or 510 nm~550 nm, or 515 nm~545 nm, or 520 nm~540 nm, or 525 nm~545 nm, or 530 nm~535 nm, preferably 530 nm;

Optionally, the center wavelength of the red light is 600-680 nm; e.g., 595-675 nm, or 590-670 nm, or 585-665 nm, or 580-650 nm, preferably 650 nm.

Optionally, the center wavelength of the near-infrared ray is 780-1100 nm; e.g., 790-1090 nm, or 800-1080 nm, or 810-1070 nm, or 820-1060 nm, or 830-1050 nm, or 840-1040 nm, or 850-1030 nm, preferably 850 nm.

Optionally, the white light illuminance is 2-6000 Lux.

Optionally, the monochromatic light illuminance is 2-500 Lux.

Optionally, the white light illuminance emitted by the lamp is 500-3000 Lux.

Optionally, the white light illuminance emitted by the lamp comprises one or more of 500 Lux, 1000 Lux, 2000 Lux and 3000 Lux.

Optionally, the display screen displays a grating image.

Optionally, the contrast ratio of the grating image is 10%-100%.

Optionally, the grating image comprises one or more of moving grating image, flickering grating image, flickering raster image and flipping grating image. Optionally, the contrast ratio of the moving grating is 10%-100%.

Optionally, the orientation of the moving grating is 0-180°.

Optionally, the spatial frequency of the moving grating image is 0.05-30 cycle/deg; e.g., 0.05-10 cycle/deg.

Optionally, the moving speed of the moving grating image is 1-10 Hz.

Optionally, the contrast ratio of the flipping grating is 10%-100%.

Optionally, the flipping frequency of the flipping grating is 20-60 Hz or 30-50 Hz, preferably 40 Hz.

Optionally, the spatial frequency of the flipping grating is 0.1-30 cpd, or 0.1 cpd.

Optionally, the contrast ratio of the flipping grating is 10%-100%, 70-90%, preferably 80%.

Optionally, the average brightness of the flipping grating is 100-500 cd/m², or 140-180 cd/m², or 150-170 cd/m², or 160 cd/m².

Optionally, the contrast ratio of the flickering raster image is 10%-100%.

Optionally, the frequency of the flickering raster image is 1-100 Hz.

Optionally, the raster size of the flickering raster image is 0.5-10 deg.

The device described in the present invention, wherein the device comprises mobile phone, tablet, or portable wearable device.

Optionally, the portable wearable device is selected from glasses, blinder, mask, hat, headband, helmet, bracelet or watch; optionally, the glasses comprise VR glasses. The device described in the present invention, wherein the portable wearable device comprises glasses frame; optionally, the portable wearable device also comprises glasses temples or hair band.

Optionally, a lamp is provided at the edge of the lens enclosed by the glasses frame.

Optionally, the lamp comprises CCFL lamp, LED lamp and/or OLED lamp.

Optionally, a display screen is provided at the center of the lens enclosed by the glasses frame. Optionally, the display screen comprises CCFL display screen, LED display screen and/or OLED display screen.

The device described in the present invention, wherein the lamp comprises monochromatic light lamp and white light lamp.

Optionally, the illuminance of the monochromatic light lamp is 2-500 lux.

Optionally, the illuminance of the white light is 2-6000 lux.

Optionally, the lamp comprises blue light lamp, green light lamp, red light lamp and white light lamp.

The device described in the present invention, wherein the display screen displays a grating image which comprises vertical moving grating, horizontal moving grating and/or flickering raster.

Optionally, the vertical moving grating is parameterized to be oriented at 0°; optionally, the spatial frequency of the vertical moving grating is 0.2 cpd; optionally, the time frequency of the vertical moving grating is 1 Hz; optionally, the contrast ratio of the vertical moving grating is 50%.

Optionally, the horizontal moving grating is parameterized to be oriented at 90°; optionally, the spatial frequency of the horizontal moving grating is 0.2 cpd; optionally, the time frequency of the horizontal moving grating is 1 Hz; optionally, the contrast ratio of the horizontal motion grating is 50%.

In another aspect, the present invention provides a use of any one of the above-mentioned devices, comprising the use for promoting the release of adenosine in an organism.

Optionally, the use comprises increasing the concentration of adenosine within an organism.

Optionally, the use comprises increasing the concentration of extracellular adenosine of neurons.

Optionally, the use comprises the treatment of neurological disease, cardiovascular disease and/or ophthalmic disease.

Optionally, the neurological disease comprises Parkinson's disease, depression, anxiety, dementia, Alzheimer's disease, post-traumatic stress disorder, hyperactivity disorder, childhood autism and/or attention deficit disorder.

Optionally, the cardiovascular disease comprises coronary heart disease and/or arrhythmia.

Optionally, the ophthalmic disease comprises myopia, strabismic amblyopia, senile retinopathy, etc.

Optionally, the use comprises the improvement of sleep disorder. Optionally, sleep disorder is improved by a visual stimulus of 20-80 Hz. Optionally, sleep disorder is improved by a visual stimulus of 30-60 Hz. Optionally, sleep disorder is improved by a visual stimulus of 35-45 Hz. Optionally, sleep disorder is improved by a visual stimulus of 40 Hz.

In another aspect, the present invention provides a method for regulating the release of adenosine in an organism, comprising the following steps:
delivering a stimulus of 1-100 Hz to a subject by using the any one of the above-mentioned devices, the delivery is a non-invasive delivery. Optionally, the stimulus comprises visual stimulus and/or acoustic stimulus.

Optionally, the frequency of the visual stimulus is 1-100 Hz; optionally, the frequency of the visual stimulus is 20-80 Hz.

Optionally, the frequency of the visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz. Optionally, the frequency of the visual stimulus comprises 30-60 Hz.

Optionally, the frequency of the visual stimulus comprises one or more of 30 Hz, 35 Hz, 40 Hz, 45 Hz, 50 Hz, 55 Hz and 60 Hz.

Optionally, the frequency of the visual stimulus comprises 35-45 Hz.

Optionally, the frequency of the visual stimulus comprises one or more of 35 Hz, 40 Hz and 45 Hz.

Optionally, the frequency of the visual stimulus is 40 Hz.

Optionally, the white light illuminance of the visual stimulus is 2000 lux or more.

Optionally, the wavelength of the monochromatic light of the visual stimulus is 480-530 nm.

Optionally, the duty ratio of the visual stimulus is 20%-80%, preferably 30%-70%, or 40%-70%, or 45-55%.

Optionally, the duty ratio of the visual stimulus is selected from one or more of 20%, 25%, 30%, 35%, 40%, 45%, 50%, 55%, 60%, 65%, 70%, 75%, and 80%.

Optionally, the duty ratio of the visual stimulus is 50%.

The method described in the present invention, wherein the visual stimulus comprises light flicker stimulus and/or image stimulus.

Optionally, the light flicker comprises white light and/or monochromatic light. Optionally, the image comprises grating image.

The method described in the present invention, wherein the monochromatic light comprises one or more of ultraviolet light, blue light, green light, red light and near-infrared ray, preferably blue light and/or green light.

Optionally, the center wavelength of the ultraviolet light is 315-400 nm. Optionally, the center wavelength of the blue light is 450-490 nm. Optionally, the center wavelength of the green light is 500 nm~560 nm. Optionally, the center wavelength of the red light is 600-680 nm. Optionally, the center wavelength of the near-infrared ray is 780-1100 nm.

Optionally, the center wavelength of the ultraviolet light is 320-395 nm, or 325-390 nm, or 330-385 nm.

Optionally, the center wavelength of the blue light is 455-488 nm, or 460-485 nm. Optionally, the center wavelength of the green light is 505 nm~555 nm, or 510 nm~550 nm, or 515 nm~545 nm, or 520 nm~540 nm, or 525 nm~545 nm, or 530 nm~535 nm.

Optionally, the center wavelength of the red light is 595-675 nm, or 590-670 nm, or 585- 665 nm, or 580-650 nm.

Optionally, the center wavelength of the near-infrared ray is 790-1090 nm, or 800-1080 nm, or 810-1070 nm, or 820-1060 nm, or 830-1050 nm, or 840-1040 nm, or 850-1030 nm.

Optionally, the center wavelength of the ultraviolet light is 380 nm; the center wavelength of the blue light is 480 nm; the center wavelength of the green light is 530 nm; the center wavelength of the red light is 650 nm; the center wavelength of the near-infrared ray is 850 nm.

Optionally, the white light illuminance is 2-6000 Lux.

Optionally, the monochromatic light illuminance is 2-500 Lux.

Optionally, the white light illuminance emitted by the lamp is 500-3000 Lux, optionally, the white light illuminance emitted by the lamp comprises one or more of 500 Lux, 1000 Lux, 2000 Lux and 3000 Lux.

The method described in the present invention, wherein the contrast ratio of the grating image is 10%-100%.

Optionally, the grating image comprises one or more of moving grating image, flickering grating image, flickering raster image and flipping grating image. Optionally, the contrast ratio of the moving grating is 10%-100%.

Optionally, the orientation of the moving grating is 0-180°.

Optionally, the spatial frequency of the moving grating image is 0.05-30 cycle/degree. Optionally, the moving speed of the moving grating image is 1-10 Hz.

Optionally, the contrast ratio of the flipping grating is 10%-100%.

Optionally, the flipping frequency of the flipping grating is 20-60 Hz or 30-50 Hz, preferably 40 Hz.

Optionally, the spatial frequency of the flipping grating is 0.1-30 cpd, or 0.1 cpd. Optionally, the contrast ratio of the flipping grating is 10%-100%, 70-90%, preferably 80%. Optionally, the average brightness of the flipping grating is 100-500 cd/m², or 140-180 cd/m², or 150-170 cd/m², or 160 cd/m².

Optionally, the contrast ratio of the flickering raster image is 10%-100%.

Optionally, the frequency of the flickering raster image is 1-100 Hz.

Optionally, the raster size of the flickering raster image is 0.5-10 degree.

In another aspect, the present invention provides an assessment method for the efficacy of a drug or a medical device, comprising analyzing the change of the concentration of adenosine within an organism.

The assessment method described in the present invention, wherein the drug or medical device is used for the treatment of neurological disease, the treatment of cardiovascular disease and/or the improvement of sleep disorder.

Optionally, the neurological disease comprises Parkinson's disease, depression, anxiety, dementia, Alzheimer's disease, post-traumatic stress disorder, hyperactivity disorder, childhood autism and/or attention deficit disorder.

Optionally, the cardiovascular disease comprises coronary heart disease and/or arrhythmia.

Optionally, the assessment method described in the present invention comprises analyzing the change in the concentration of extracellular adenosine of neurons.

The beneficial effects of the present invention comprise:
(1) the present invention provides a novel strategy for regulating the release of adenosine with the potential advantages of being non-invasive, low side effects, good tolerance and good efficacy.
(2) the present invention provides a novel strategy for improving sleep disorders, which promotes sleep by adenosine with a relatively small action scope on the neural center of the cerebrum, a mild effect, good relative safety and a low probability of side effects on neural center.
(3) the present invention provides a portable wearable device, which is convenient for subjects to use it anytime and anywhere.

### Brief description of the drawings

FIG. 1 shows the promotion effect of the 40 Hz light flicker illumination on the total concentration of adenosine in the visual primer cortex (V1) in Example 1 analyzed by ultra-high performance liquid chromatography.

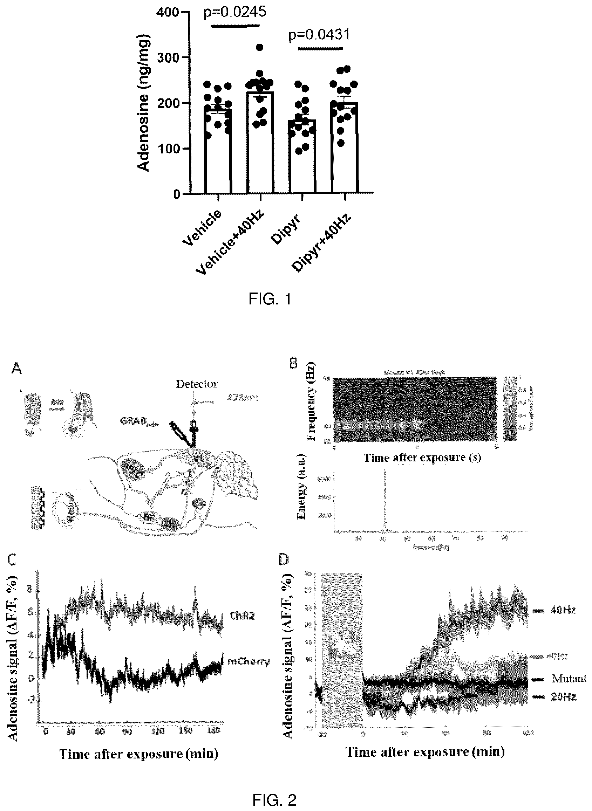

FIG. 2 shows the effect of the light flicker stimuli at different frequencies on the release of adenosine in the V1 area in Example 1; A, the schematic diagram of the experiment for detecting the adenosine signal in the V1 after light flicker stimulation; B, the 40 Hz light flicker can effectively induce gamma oscillations in the V1; C, the positive neurons activated optogenetically (40 Hz) in the V1 (Parvalbumin, PV) effectively increase the release of adenosine; D, the time-adenosine release corresponding curve in V1 under light flicker stimulation at different frequencies (20 Hz, 40 Hz, 80 Hz) (n=3-5/group).
FIG. 3 shows the effect of the light flicker stimulus in Example 2 on the concentrations of the neuromodulators 5-hydroxytryptamine and acetylcholine in the brain region of visual primer cortex (V1).
FIG. 4 shows the brain region specificity of the adenosine release increased by the 40 Hz light flicker in Example 3. A, the time-adenosine concentration change curves of 8 brain regions such as basal forebrain (BF), visual primer cortex (V1), Nucleus accumbens (NAc), Ventral tegmentalnucleus (VTA), Superior colliculus (SC), Medial prefrontal cortex (mPFC), Hippocampus (HPC) and Dorsomedial striatum (DMS) after 30 min stimulation by the 40 Hz light flicker.

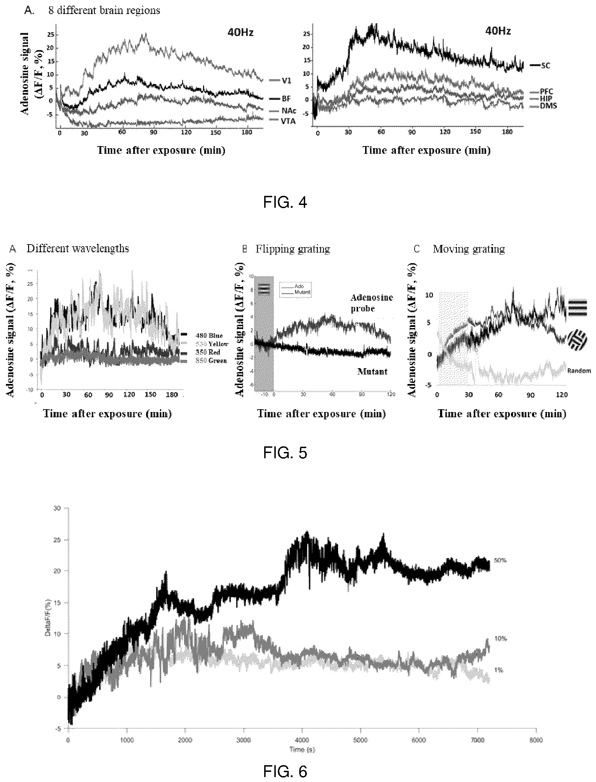

FIG. 5 shows extracellular adenosine specificity in the visual cortex increased by different visual stimulation modes in Example 4. A, the time-concentration corresponding curve of the adenosine release in the V1 by 40 Hz light flicker stimuli at different wavelengths; B, the visual stimulus of the flipping gratings or sinusoidal gratings can significantly increase the release of adenosine in V1; n=3-5 per group in A-C; C, the time-concentration corresponding curve of the adenosine release in the V1 region of visual cortex in different stimulation modes of the moving grating, where the circular legend indicates that the release of adenosine in the V1 of visual cortex increases rapidly with the stimulation in the moving grating stimulation mode under vertical and orthogonal inhibition, the square legend indicates that the release of adenosine in the V1 region of visual cortex increases slowly in the full-screen moving grating stimulation mode at the orientation of 90°, and Random indicates that there is no increase in the release of adenosine in the full-screen moving grating stimulation at random orientation. The shaded part corresponds to the visual stimulation time, n=3 per group.
FIG. 6 shows the effect of the 40 Hz strobe at different duty ratios on extracellular adenosine in the visual cortex in Example 5.
FIG. 7 shows the effect of the 40 Hz strobe with different illuminance on extracellular adenosine of the visual cortex in Example 6.
FIG. 8 shows the effect of the 40 Hz strobe at different light wavelength on extracellular adenosine of the visual cortex in Example 6.

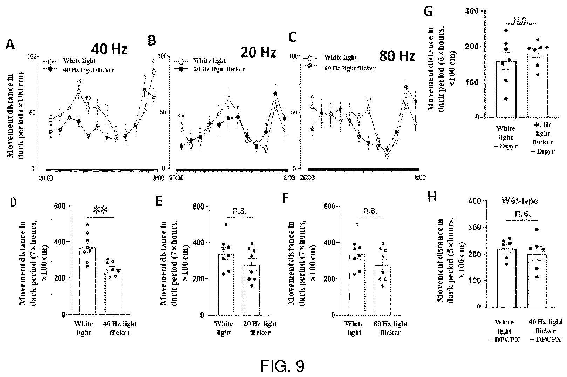

FIG. 9 shows the significant reduction in the nocturnal movement activity of mice by 40 Hz via adenosine signal in Example 7; A-C, the time-movement amount corresponding curves of mice during the nighttime after 30 minutes of normal white light and the light flickers at 20 Hz, 40 Hz and 80 Hz; D-F, the statistical analysis diagram of the movement amount of mice in the first 7 hours of the nighttime after 30 minutes of normal white light and the light flickers at 20 Hz, 40 Hz and 80 Hz; G. the intraperitoneal injection of the equilibrate nucleotide transporter (ENT) inhibitor Dipyridamole (Dipyr, 15mg/kg) blocks the movement inhibition effect of the 40 Hz light flicker in CD73-KO mice; H, the intraperitoneal injection of the active antagonist DPCPX (6mg/kg) of the adenosine A1 receptor blocks the movement inhibition effect induced by the 40 Hz light flicker. n=8/group, * p<0.05, **p<0.01 v normal white light group.

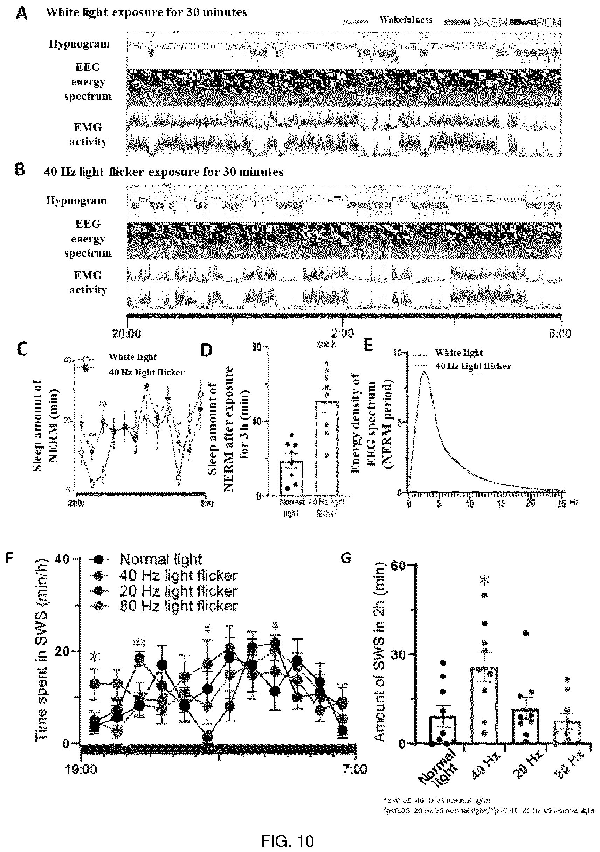

FIG. 10 shows the significant increase of the nocturnal sleep amount in mice by the 40 Hz light flicker in Example 8; A-B, the schematic diagrams of the electroencephalography and electromyography spectra of mice during the nighttime after light treatment in the last 30 minutes of the daytime phase; C, the time-sleep amount corresponding curve of the nocturnal sleep amount of mice significantly increased by the 40 Hz light flicker; D, the statistical chart of the sleep amount in the first 3 hours of the dark phase after the light treatment; E, the spectral density distribution of the sleep energy of mice during the nighttime after the light treatment shows that the 40 Hz light flicker increases the sleep amount of mice without affecting the spectral energy density; n=7, **p<0.01, ***p<0.001, vs normal white light group; F-G: the light flicker treatments of 20 Hz and 80 Hz do not significantly increase the sleep amount of mice.
FIG. 11 shows the concentration-dependent increase of the total sleep amount of mice by the adenosine injection into the visual cortex in Example 8.
FIG. 12 shows the distribution of the tolerance scores in Example 9.
FIG. 13 shows the improvement on various sleep parameters of the patients with chronic insomnia, comprising the sleep latency, total sleep time, wakefulness after sleep onset, etc., by the 40 Hz light flicker in Example 10.
FIG. 14 shows the significant reduction of 40% in astrocyte reactivity compared to the control side (AAV2/9-GfaABC1D-mCherry) after the foot shock in Example 11, indicating that the AAV2/9-GfaABC1D-mCherry-hPMCA2w/b virus can indeed reduce the activity of astrocytes.
FIG. 15 shows the effect of the 40 Hz light flicker stimulus on the concentration of extracellular adenosine of the astrocytes in Example 11.
FIG. 16 shows the effect of the 40 Hz light flicker stimulus on the concentration of extracellular adenosine of the neurons in Example 11.
FIG. 17 shows the schematic diagram of the portable visual stimulation device in Example 12.
FIG. 18 shows the schematic diagram of the stimulation mode of the moving grating, with alternating black and white stripes, moving in the direction of the arrow in the figure.
FIG. 19 shows the schematic diagram of the image change of the flipping grating: the local light and dark of the grating (taking dashed lines as an example) alternate at a frequency of 40 Hz; the screen refresh rate is 120 Hz.
FIG. 20 shows the schematic diagram of the flickering raster.

### Detailed description of the invention

As described above, the objective of the present invention is to provide a device for regulating the release of adenosine in an organism to solve the technical problem that at present there is no safe and effective strategy for increasing the concentration of adenosine in an organism or precisely improving sleep disorder.

The technical solution of the present invention will be described clearly and completely as follows. Obviously, based on the specific embodiments in the present invention, all other embodiments obtained by those of ordinary skilled in the art without making creative labor fall within the scope of protection of the present invention.

It should be noted that the terms used herein are intended only to describe specific embodiments and are not intended to limit the exemplary embodiments according to the present invention.

Cold Cathode Fluorescent Lamp (CCFL) has the advantages such as high power and high brightness, and is widely used in display screen, lighting and other fields. Light-emitting diode (LED) is a commonly used light-emitting device.

Organic Light-Emitting Diode (OLED) belongs to a current-type organic light-emitting device.

The present invention provides a device for regulating the release of adenosine in an organism, comprising a stimulus generator for generating a sound of 1-100 Hz and/or for generating a light flicker of 1-100 Hz and/or a full-screen visual stimulus. The stimulus generator may comprise, e.g., light flicker stimulus (which can be emitted by strobe or grating device) generator, visual stimulus generator and/or acoustic stimulus generator. Optionally, the visual generator emits a visual stimulus of 20-80 Hz, e.g., a visual stimulus of 30-60 Hz can be emitted, e.g., a visual stimulus of 35-45 Hz can be emitted, e.g., a visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz can be emitted. Optionally, the frequency of the visual stimulus is 40 Hz. Optionally, the white light illuminance of the visual stimulus is 2000 lux or more; optionally, the wavelength of the monochromatic light of the visual stimulus is 480-530 nm; optionally, the duty ratio of the visual stimulus is 20%-80%, preferably 30%-70%, or 40%-70%, or 45-55%; optionally, the duty ratio of the visual stimulus is selected from one or more of 20%, 25%, 30%, 35%, 40%, 45%, 50%, 55%, 60%, 65%, 70%, 75%, and 80%; optionally, the duty ratio of the visual stimulus is 50%. The visual stimulus generator comprises a lamp and/or a display screen; optionally, the lamp comprises CCFL lamp, LED lamp and/or OLED lamp; optionally, the display screen comprises CCFL display screen, LED display screen, and/or OLED display screen.

According to the device provided in the present invention, wherein the stimulus generator may emit white light and/or monochromatic light; optionally, the monochromatic light comprises one or more of ultraviolet light, blue light, green light, red light and near-infrared ray; optionally, the center wavelength of the ultraviolet light is 315-400 nm, 320-395 nm, or 325-390 nm, or 330-385 nm; preferably 380 nm; the center wavelength of the blue light is 450-490 nm, or 455-488 nm, or 460-485 nm; preferably 480 nm; the center wavelength of the green light is 500 nm~560 nm, or 505 nm~555 nm, or 510 nm~550 nm, or 515 nm~545 nm, or 520 nm~540 nm, or 525 nm~545 nm, or 530 nm~535 nm; preferably 530 nm; the center wavelength of the red light is 600-680 nm, or 595-675 nm, or 590-670 nm, or 585-665 nm, or 580-650 nm; preferably 650 nm; the center wavelength of the near-infrared ray is 780-1100 nm, or 790-1090 nm, or 800-1080 nm, or 810-1070 nm, or 820-1060 nm, or 830-1050 nm, or 840-1040 nm, or 850-1030 nm; preferably 850 nm; optionally, the white light illuminance is 2-6000 Lux, and the monochromatic light illuminance is 2-500 Lux. Optionally, the white light illuminance emitted by the lamp is 500-3000 Lux, optionally, the white light illuminance emitted by the lamp comprises one or more of 500 Lux, 1000 Lux, 2000 Lux and 3000 Lux.

Light of different colors and different bands will produce different stimulating effects, which can be used to treat different diseases by adjusting the color, wavelength, frequency and other parameters of the light emitted by the device.

According to the device provided in the present invention, the stimulus generator can emit a grating image stimulus; optionally, the contrast ratio of the grating image is 10%-100%; optionally, the grating image comprises one or more of moving grating image, flickering grating image, flickering raster image and flipping grating image; optionally, the contrast ratio of the moving grating is 10%-100%; optionally, the orientation of the moving grating is 0-180°; optionally, the spatial frequency of the moving grating image is 0.05-30 cycle/deg; e.g., 0.05-10 cycle/deg; optionally, the moving speed of the moving grating image is 1-10 Hz;
optionally, the moving grating may comprise vertical moving grating, or may comprise horizontal moving grating. The parameters of the vertical moving grating are the orientation of 0°; the spatial frequency of 0.2 cpd, the time frequency of 1 Hz and the contrast ratio of 50%; the parameters of the horizontal moving grating are the orientation of 90°; the spatial frequency of 0.2 cpd, the time frequency of 1 Hz and the contrast ratio of 50%. Optionally, the contrast ratio of the flickering raster image is 10%-100%; optionally, the frequency of the flickering raster image is 1-100 Hz; optionally, the raster size of the flickering raster image is 0.5-10 deg.

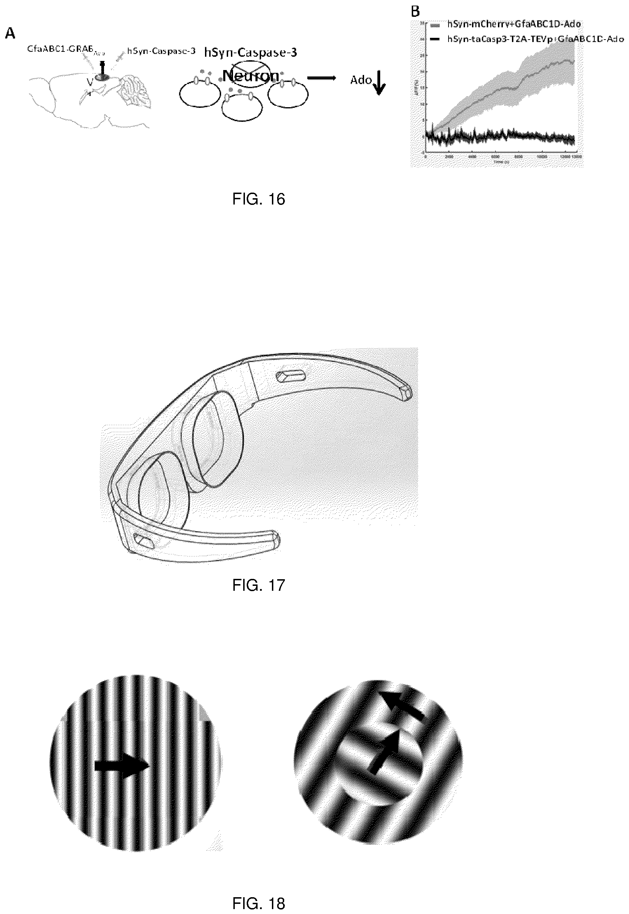

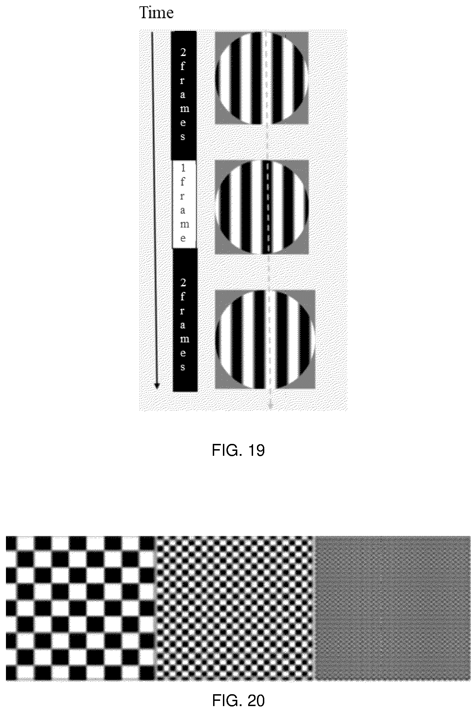

As shown in FIG. 17-19, the present invention provides different gratings. FIG. 17 is the schematic diagram of the stimulation mode of the moving grating provided in the present invention. The variable parameters comprise: 100-500 candelas per square meter (cd/m²), orientation (0-180°), spatial frequency (0.2-30 cycle/degree, i.e., cpd, the width of a single grating), time frequency (1-10 Hz, the speed of grating motion), contrast ratio (1-100%), range size of stimulation (0-30° or full-screen stimulation), orthogonal stimulation (central receptive field of 5°, peripheral receptive field of 15°). FIG. 18 is the schematic diagram of the stimulation mode of the flipping grating provided in the present invention. The variable parameters comprise: 100-500 candelas per square meter (cd/m²), orientation (0-180°), spatial frequency (0.1-30 cycle/degree, i.e., cpd, the width of a single grating), flickering frequency (1-100 Hz), contrast ratio (1-100%), range size of stimulation (0-30° or full-screen stimulation). FIG. 19 is the schematic diagram of the stimulation mode of the flickering raster provided in the present invention, where the display screen is presented in a chess-board raster mode. The variable parameters: single black/white raster as length and width (1°~5°), contrast ratio (1%-100%), flickering frequency (1~100Hz). Wherein, contrast ratio refers to the measurement of different brightness levels between the brightest white and the darkest black in the bright and dark areas in an image. The greater the difference range, the greater the contrast ratio (e.g., contrast ratio of 100%) and the smaller the difference range, the smaller contrast ratio (e.g., contrast ratio of 10%). The higher the contrast ratio, the easier it is for our eyes to distinguish.

The present invention provides a device for regulating the release of adenosine in an organism, wherein the device comprises mobile phone, tablet, or portable wearable device. Wherein the portable wearable device can be glasses, blinder, mask, hat, headband, helmet, bracelet or watch; optionally, the glasses comprise VR glasses. Optionally, the portable wearable device comprises glasses frame; optionally, the portable wearable device also comprises glasses temples or hair band; optionally, a lamp is provided at the edge of the lens enclosed by the glasses frame; optionally, the lamp comprises CCFL lamp, LED lamp and/or OLED lamp; optionally, a display screen is provided at the center of the lens enclosed by the glasses frame; optionally, the display screen comprises CCFL display screen, LED display screen, and/or OLED display screen.

The device provided in the present invention can be used to promote the release of adenosine in an organism; for example, it is used to increase the concentration of adenosine within an organism; specifically, it is used to increase the concentration of extracellular adenosine of neurons.

It has been widely studied and recognized that adenosine and its receptors can be used to treat many diseases of the neurological system and the cardiovascular system, wherein the neurological disease comprises Parkinson's disease, depression, anxiety, dementia, Alzheimer's disease, post-traumatic stress disorder, hyperactivity disorder, childhood autism, attention deficit disorder, etc.; the cardiovascular disease comprises coronary heart disease, arrhythmia, etc. Since the device of the present invention can effectively increase the concentration of adenosine in the brain of an organism, it is expected that the device of the present invention can also be used to treat many diseases of the neurological system and the cardiovascular system.

The device of the present invention can be used to treat neurological diseases, cardiovascular diseases and/or ophthalmic diseases; optionally, the neurological disease comprises Parkinson's disease, depression, anxiety, dementia, Alzheimer's disease, post-traumatic stress disorder, hyperactivity disorder, childhood autism, attention deficit disorder, etc.; optionally, the cardiovascular disease comprises coronary heart disease, arrhythmia, etc.; optionally, the ophthalmic disease comprises myopia, strabismic amblyopia, senile retinopathy, etc. The device of the present invention can be used to improve sleep disorder, optionally, the sleep disorder can be improved by a visual stimulus of 20-80 Hz, the sleep disorder can be improved by a visual stimulus of 30-60 Hz, optionally, the sleep disorder can be improved by a visual stimulus of 35-45 Hz, optionally, the sleep disorder can be improved by a visual stimulus of 40 Hz.

The present invention provides a method for regulating the release of adenosine in an organism, comprising the following steps:
delivering the stimulus of 1-100 Hz to the subject using the device provided in the present invention, wherein the delivery is non-invasive delivery; optionally, the stimulus comprises visual stimulus and/or acoustic stimulus. Optionally, the frequency of the visual stimulus is 1-100 Hz, optionally, the frequency of the visual stimulus is 20-80 Hz, e.g., 30-60 Hz, e.g., 35-45 Hz; the frequency of the visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz. Optionally, the frequency of the visual stimulus is 40 Hz. Optionally, the white light illuminance of the visual stimulus is 2000 lux or more; optionally, the wavelength of the monochromatic light of the visual stimulus is 480-530 nm; optionally, the duty ratio of the visual stimulus is above 10%; optionally, the duty ratio of the visual stimulus is 20%-80%, preferably 30%-70%, or 40%-70%, or 45-55%; optionally, the duty ratio of the visual stimulus is selected from one or more of 20%, 25%, 30%, 35%, 40%, 45%, 50%, 55%, 60%, 65%, 70%, 75%, and 80%; optionally, the duty ratio of the visual stimulus is 50%.

Optionally, the method for regulating the release of adenosine in an organism, wherein the visual stimulus comprises light flicker stimulus and/or image stimulus; optionally, the light flicker comprises white light and/or monochromatic light; optionally, the image comprises grating image. Optionally, the monochromatic light comprises one or more of ultraviolet light, blue light, green light, red light and near-infrared ray; optionally, the center wavelength of the ultraviolet light is 315-400 nm; optionally, the center wavelength of the ultraviolet light is 320-395 nm, or 325-390 nm, or 330-385 nm; the center wavelength of the blue light is 450-490 nm; optionally, the center wavelength of the blue light is 455-488 nm, or 460-485 nm; the center wavelength of the green light is 500 nm~560 nm; optionally, the center wavelength of the green light is 505 nm~555 nm, or 510 nm~550 nm, or 515 nm~545 nm, or 520 nm~540 nm, or 525 nm~545 nm, or 530 nm~535 nm; the center wavelength of the red light is 600-680 nm; optionally, the center wavelength of the red light is 595-675 nm, or 590-670 nm, or 585- 665 nm, or 580-650 nm; the center wavelength of the near-infrared ray is 780-1100 nm; optionally, the center wavelength of the near-infrared ray is 790-1090 nm, or 800-1080 nm, or 810-1070 nm, or 820-1060 nm, or 830-1050 nm, or 840-1040 nm, or 850-1030 nm. Optionally, the center wavelength of the ultraviolet light is 380 nm; the center wavelength of the blue light is 480 nm; the center wavelength of the green light is 530 nm; the center wavelength of the red light is 650 nm; the center wavelength of the near-infrared ray is 850 nm; optionally, the white light illuminance is 2-6000 Lux, and the monochromatic light illuminance is 2-500 Lux. Optionally, the white light illuminance emitted by the lamp is 500-3000 Lux, optionally, the white light illuminance emitted by the lamp comprises one or more of 500 Lux, 1000 Lux, 2000 Lux and 3000 Lux. Optionally, the contrast ratio of the grating image is 10%-100%; optionally, the grating image comprises one or more of moving grating image, flickering grating image, flickering raster image and flipping grating image; optionally, the contrast ratio of the moving grating is 10%-100%; optionally, the orientation of the moving grating is 0-180°; optionally, the spatial frequency of the moving grating image is 0.05-30 cycle/degree; optionally, the moving speed of the moving grating image is 1-10 Hz; optionally, the contrast ratio of the flickering raster image is 10%-100%; optionally, the frequency of the flickering raster image is 1-100 Hz; optionally, the raster size of the flickering raster image is 0.5-10 degree. Optionally, the method for regulating the release of adenosine in an organism of the present invention can increase the concentration of extracellular adenosine of neurons. The present invention provides an assessment method for the efficacy of a drug or a medical device, comprising analyzing the change of the concentration of adenosine within an organism (e.g., brain regions such as visual cortex (V1), basal forebrain (BF), superior colliculus (SC), and/or in and out of cells such as neurons). The drug or medical device is used for the treatment of neurological disease, the treatment of cardiovascular disease and/or the improvement of sleep disorder; optionally, the neurological disease comprises Parkinson's disease, depression, anxiety, dementia, Alzheimer's disease, post-traumatic stress disorder, hyperactivity disorder, childhood autism and/or attention deficit disorder; optionally, the cardiovascular disease comprises coronary heart disease and/or arrhythmia.

The adenosine regulatory device and sleep disorder improvement solution of the present invention are described below by specific examples.

### Example 1. The 40 Hz light flicker stimulus frequency-dependently activates the concentration of adenosine in the V1 region

### 1) Ultra-high performance liquid chromatography (UPLC) analysis shows that the 40 Hz light flicker illumination can significantly increase the total concentration of adenosine in the V1

The total concentration of adenosine in the visual cortex of mice was detected by ultra-high performance liquid chromatography (which is reported including 95% extracellular adenosine and 5% intracellular adenosine in literature). Dipyridamole (Dipyr) (a 6 mg/kg adenosine transporter inhibitor) dissolved in 0.5% methylcellulose was intraperitoneally injected 30 minutes before illumination, while the control group was given 0.5% methylcellulose. 30 minutes after the injection, the blank control group and the Dipyridamole group were illuminated for 30 minutes by the light flicker stimulus with the illuminance of 3000 lux, the duty ratio of 50% and the strobe of 40 Hz (referred to as "40Hz light flicker stimulus" hereinafter). After illumination, the materials were sampled, weighed and rapidly frozen in liquid nitrogen immediately. After the sample processing, the peak value at about 2.0 was detected on machine with reference to the parameters of the standard substance, and the area under the curve at this moment was taken as the concentration of adenosine in the sample. Finally, the concentration of adenosine was obtained after checking the sample quality and calibrating.

As shown in Figure 1, it is found that after light flicker stimulation, the concentration of adenosine significantly increases (p=0.0245, n=7), while Dipyridamole can effectively inhibit the total concentration of intracellular and extracellular adenosine; 30 minutes after illumination of the 40 Hz light flicker, the inhibition of the total concentration of intracellular and extracellular adenosine by Dipyridamole is effectively reversed (p=0.0431, n=7). It indicates that illumination of the 40 Hz light flicker for 30 minutes can significantly increase the concentration of adenosine in the V1 region and also increase the concentration of adenosine in the Dipyridamole group.

### 2) Frequency dependence

In this study, a novel genetically encoded adenosine probe with high specificity and high time resolution was employed to study adenosine kinetic changes in different brain regions at different frequencies. The novel adenosine probe works by measuring the changes of the concentration of extracellular adenosine through the fluorescence intensity of green fluorescent protein when the G protein-coupled receptor for adenosine of the cell is activated. As shown in Figure 2A, we injected the adenosine probe (AAV2/9-hsyn-GRABAdo1.0, BrainVTA (Wuhan) Co., Ltd., PT-1348) and the adenosine probe control mutant (AAV2/9-hsyn-GRABAdo-mut, BrainVTA (Wuhan) Co., Ltd., PT-1348) virus into the visual primer cortex (V1) brain region of mice with reference to the stereotaxic coordinates (3.6 µm anterior, ±2.4 µm lateral, -0.6 µm deep). After captivity for 3 weeks, the mice were exposed to a light flicker environment of 20 Hz, 40 Hz and 80 Hz (12.5 ms light on, 12.5 ms light off, i.e., the duty ratio of 50%, 3000 lux, 60 watts) for 30 min, and the changes of the adenosine concentration before and after light flicker exposure were compared.

The results are shown in Fig. 2. As shown in Fig. 2B, 40 Hz light flicker can effectively induce γ oscillation in the V1 region. As shown in Figure 2C, the change of the adenosine concentration significantly increases after illumination of the 40 Hz light flicker stimulus. Figure 2D shows the corresponding curves of time-adenosine release in the V1 region under light flicker stimuli at different frequencies (20 Hz, 40 Hz, 80 Hz) (n=3-5/ group). The mice show a slight increase in the adenosine signal under a 80 Hz light flicker stimulus, but no change in the concentration of adenosine at 20 Hz, indicating that 40 Hz light flicker frequency-dependently activated cells in the V1 region to release adenosine. In conclusion, the release of extracellular adenosine may be related to the specific illumination frequency, which will provide an important neurochemical basis for exploring visual stimuli to improve sleep and cognitive disorder, etc.

### Example 2. The light flicker stimulus specifically changes adenosine but does not change the neuromodulators 5-hydroxytryptamine and acetylcholine in the brain region of visual primer cortex (V1)

Neurotransmitters and neuromodulators are the key mediator molecules in the information communication between neurons for effective information transfer and integration. In order to verify whether the 40 Hz light flicker stimulus specifically changes adenosine in the brain region of visual primer cortex (V1), we detected the dynamic changes of 5-hydroxytryptamine (5-HT) and acetylcholine (ACh) under the 40 Hz light flicker stimulus using a genetically encoded fluorescent probe combined with an optical fiber recording system, respectively.

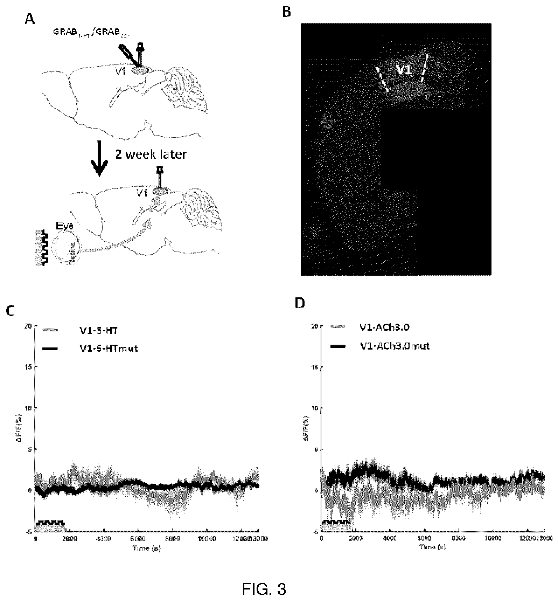

We injected AAV9-hsyn-GRAB_{5-HT3.5} and AAV9-hsyn-GRAB_{5HT2.1-mut}, AAV9-hsyn-GRAB_{ACh3.0} and AAV9-HSYN-Grab_{ACh3.0-mut} into the left and right sides of the visual cortex of mice, respectively (FIG. 3A-B). After virus expression for 2 weeks, the intervention of 40 Hz light flicker was performed and the concentration changes of 5-hydroxytryptamine and acetylcholine were recorded in real time. As the results shown in Figure 3C-D, there are no significant changes in either 5-HT or Ach in the visual cortex (V1) after 40 Hz light flicker stimulation.

### Example 3. The 40 Hz light flicker stimulus can effectively improve the release levels of adenosine in the brain regions such as V1, BF, mPFC and SC, which is brain region-specific

In this study, a novel genetically encoded adenosine probe was employed to detect the release levels of adenosine in the brain regions of visual cortex (V1), basal forebrain (BF), nucleus accumbens (NAc), ventral tegmentalnucleus (VTA), superior colliculus (SC), medial prefrontal cortex (mPFC), hippocampus (HPC) and dorsomedial striatum (DMS). As shown in FIG. 4, the adenosine signals were recorded after 30 min of giving the 40 Hz light flicker stimulus. We found that the release levels of adenosine in the brain regions such as V1, SC, BF, mPFC and HPC significantly increased, while no increase in the signals of adenosine was found in the control group injected with AAV2/9-hsyn-Mut virus (BrainVTA (Wuhan) Co., Ltd., PT-1348). Among the brain regions with increased adenosine signal, the adenosine signals were strongest in the brain regions related to visual information encoding, such as V1 and SC; the adenosine signals in the brain regions related to sleep-wake such as BF were also enhanced obviously; the adenosine signals in the brain regions related to cognition such as mPFC and HPC were slightly enhanced. These results suggest that the non-invasive stimulation mode of the 40 Hz light flicker can be used to increase the levels of adenosine in the sleep-related brain regions to regulate sleep.

### Example 4. Different visual stimulation modes increase the release levels of adenosine in the V1

### 1) The 40 Hz light flicker stimulus

The differences of the release levels of adenosine in the V1 region under the 40 Hz light flickering stimuli at different wavelengths are shown in FIG. 5A. Only the blue light at the wavelength of 480 nm and the green light at the wavelength of 530 nm can significantly increase the concentration of adenosine in the V1 region, while the light flicker stimuli at other wavelengths (380 nm, 650 nm, 850 nm, etc.) do not increase the concentration of adenosine in the V1 region.

### 2) The effect of the moving grating

As shown in Figure 5C, the horizontal moving grating stimulus was used (left panel of FIG. 18, arrow indicates the direction of motion). The stimulation parameter was as follows: the grating was displayed in full screen at a high contrast ratio (80%) (the average brightness of the screen was 160 cd/m²), so as to ensure that the mice could see the stimuli throughout the experiment. The spatial frequency of the horizontal moving grating was 0.2 cpd, and the time frequency was 1-10 Hz (i.e., the grating moving speed). Meanwhile, the level changes of adenosine in the V1 region of mice were detected under the stimulus combination formed by the 5° horizontal moving grating in the center and the 15° vertical moving grating in the periphery (center-periphery vertical moving grating, FIG. 18 therefrom).

The results show that both moving gratings can significantly increase adenosine in the V1, but the horizontal moving grating shows a smaller increase than the center-periphery vertical moving grating (FIG. 5C, the ΔF/F value is 5% in the horizontal moving grating and 8% in the center-peripheral vertical moving grating), indicating that the wide-range grating stimulus has an important effect on the production of extracellular adenosine.

### 3) The effect of the vertical flipping grating

As shown in FIG. 5B, the vertical flipping grating stimulus at the frequency of 40 Hz is employed under the condition that the light intensity remained unchanged. The stimulation parameters were: the vertical sinusoidal flipping grating with the spatial frequency of 0.1 cpd and the flipping frequency of 40 Hz (i.e., a black grating and a white grating flicker alternately, FIG. 19), and there was no change in the average light intensity in a specific time cycle (eliminating the luminance nonlinearity of the screen by Gamma-correction). The grating was displayed full screen at a high contrast ratio (80%) (the average screen brightness was 160 cd/m²) to ensure that the mice could see the stimulus throughout the experiment.

The results show that the vertical flipping grating can significantly increase adenosine in the V1, but to a lesser extent than the 40 Hz light flickering stimulus (Figure 5B, the ΔF/F value is 5% in the flipping grating and 15-20% in the 40 Hz light flicker stimulus), indicating that the light intensity has an important effect on the production of extracellular adenosine.

### Example 5. Effects of the 40 Hz light flicker stimuli at different duty ratios on extracellular adenosine in the visual cortex

Considering the effect of the illuminance (total energy) of the white light flicker stimulus on the concentration of extracellular adenosine, and different total energy produced by the strobe of 40 Hz (same illuminance of 3000 lux) at different duty ratios (1%, 10%, 50%), therefore, we detected the effects of the stimulation of the strobe of 40 Hz (same illuminance of 3000 lux) for 30 minutes on the levels of adenosine in the V1 region at different duty ratios (1%, 10%, 50%).

The results are shown in FIG. 6, it can be seen that the peak ΔF/Fs of the levels of the adenosine increase in the V1 are at 5 and 10 at the duty ratio of 1% and the duty ratio of 10%, respectively, which are lower than that of the stimulus at the duty ratio of 50% (the peak ΔF/F is 25), indicating that the white light flicker stimulus can significantly increase the level of adenosine in the V1 at the duty ratio of 50%. Therefore, under the same illuminance (3000 lux), the white light flicker stimulus at the frequency of 40 Hz increases the concentration of extracellular adenosine in a duty ratio (1%, 10%, 50%)-dependent (specific) manner. When the duty ratio is 50%, the strobe of 40 Hz increases the level of adenosine in the V1 most significantly.

### Example 6. Effects of the strobe of 40 Hz with different light intensities and light wavelengths on extracellular adenosine in the visual cortex

### 1) The effect of the light intensity

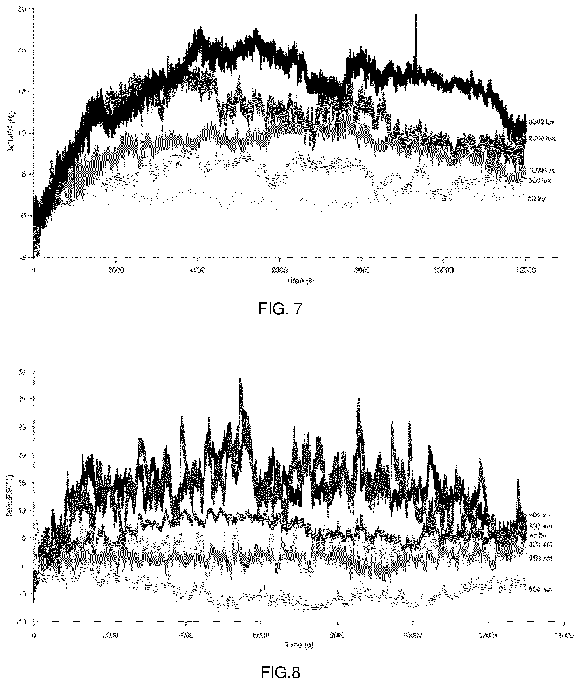

The white light flicker stimuli of 40 Hz with different illuminance (50 Lux, 500 Lux, 1000 Lux, 2000 Lux and 3000 Lux) were employed to study the effects of different illuminance on the production of adenosine in the visual cortex. As shown in Figure 7, we found that the 40 Hz light flicker with low light intensity (500 Lux) induced low level of adenosine, and the peak ΔF/F of the level of adenosine increased by the white light flicker stimulus of 40 Hz with high light intensity (3000 Lux) was at 20. Moreover, all 40 Hz light flicker stimuli with illuminance of 2000 or more Lux significantly increased the concentration of adenosine in the V1 region. In conclusion, these studies about white light from different illuminance and vertical gratings increasing adenosine in the V1 region suggest that the light intensity is a key factor in producing the levels of extracellular adenosine in the visual primer cortex of the brain.

### 2) The effect of light wavelength

As shown in Figure 8, under the 40 Hz monochromatic light flicker stimulation with the illuminance of 500 Lux, the blue light at the wavelength of 480 nm and the green light at the wavelength of 530 nm can significantly increase the concentration of adenosine in the V1 region under the strobe of 40 Hz, while the light flicker stimuli at other wavelengths (380nm, 650nm, 850nm, etc.) do not increase the concentration of adenosine in the V1 region. It is notably that the dynamics of this increase in the concentration of adenosine in the V1 by the single wavelength are basically similar (increasing and decreasing from 10 minutes, reaching the highest concentration at 30 minutes, and lasting for 2-3 hours).

### Example 7. The 40 Hz light flicker stimulus inhibits the nocturnal movement activity of mice

Based on our previous work, we had found that the 40 Hz light flicker stimulus could significantly increase the concentration of adenosine in the BF region, which plays an important role in the sleep-wake regulation. Thus, we speculated that the 40 Hz light flicker stimulus can regulate sleep rhythm. We firstly exposed the wild-type C57BL/6J mice to light flickers at different frequencies in the last 30 minutes of the daytime phase (19:30-20:00), and then monitored the nocturnal movement activity of the mice, thereby reflecting the sleep-wake states of the mice indirectly. As shown in FIG. 9, the movement activity of mice is inhibited for as long as 8 hours after 30 min of giving the 40 Hz light flicker stimulus; however, both 20 Hz and 80 Hz light flicker stimuli have no significant effect on the movement activity of mice, indicating that light flicker stimulus inhibiting the nocturnal movement activities of mice is frequency-specific. To further confirm the origin of adenosine produced by the 40 Hz light flicker stimulus, we firstly employed CD73-KO mice to conduct the 40 Hz illuminance experiment, and found that the 40 Hz light flicker stimulus still significantly reduced the nocturnal movement activity of CD73-KO mice, indicating that the extracellular ATP metabolism pathway is not the main origin of adenosine in this phenotype. We then intraperitoneally injected CD73-KO mice with the nucleotide transporter inhibitor Dipyridamole (Dipyr) at 15mg/kg. It was found that the movement inhibition effect induced by the 40 Hz light flicker completely disappeared, suggesting that intracellular adenosine transport via nucleotide transporters to outside the cell is the main mechanism of the adenosine release stimulated by the 40 Hz light flicker in this phenotype. Meanwhile, we also intraperitoneally injected CD73-KO mice with the adenosine A1 receptor activity antagonist 8-Cyclopentyl-1,3-dipropyl-1H-purine-2,6(3H,7H)-dione (DPCPX) at 6 mg/kg. It was found that DPCPX could block the movement inhibition effect caused by the 40 Hz light flicker, either.

### Example 8. The 40 Hz light flicker stimulation for 30 min can significantly increase the sleep amount of mice by enhancing the extracellular adenosine signal in the cortex

1) The 40 Hz light flicker of 30 min can significantly increase the sleep amount of mice In order to verify the regulatory effect of the 40 Hz light flicker on the sleep of mice, we used a polysomnographic system to accurately determine and count the sleep-wake states of mice according to the characteristics of different time phases of sleep-wake by recording the electroencephalogram and electromyogram of mice. In this experiment, we used an self-controlled method. On the first day, the mice were given normal white light in the last 30 minutes of the daytime phase, after which the sleep amount during the nighttime was recorded and analyzed. On the second day, the mice were given the 40 Hz light flicker in the last 30 minutes of the daytime phase, after which the sleep amount during the nighttime was recorded and analyzed. As shown in FIG. 10A-E, by comparing the sleep amount of mice during the nighttime on both days, we found that the 40 Hz light flicker could significantly increase the NREM amount of mice during the nighttime without affecting the slow-wave activity in NREM sleep. These results suggest that the 40 Hz light flicker is a good, non-invasive sleep promoter which promotes physiologic sleep.
2) The light flicker stimulus significantly increasing the sleep amount of mice is also frequency-specific
   We further demonstrated that the sleep-promoting effect of the light flicker was frequency-specific. At first, the basal electroencephalogram (EEG)/electromyogram (EMG) of mice throughout the dark phase (19:00 pm-7:00 am) after normal illumination during daytime were recorded on day 1. Then the mice were exposed to the light flicker stimuli at three different frequencies (20 Hz, 40 Hz, and 80 Hz; 3000 lux; duty ratio of 50%) in the last 30 minutes of the illumination phase on days 2, 9 and 16 (6:30-7:00 pm of the illumination phase which was from 7:00 am to 19:00 pm) for 30 minutes, and the EEG/EMG was recorded during the subsequent dark phase. By comparing the sleep amount during the dark phase after different treatments, it is found that the 40 Hz light flicker of 30 minutes presents a strong sleep-promoting effect on mice. Such significant sleep-promoting effect occurs mainly in the first 2 hours. Importantly, the 20 Hz and 80 Hz light flicker treatments do not increase the sleep amount significantly (FIG. 10F-G), demonstrating that the sleep-promoting effect of the light flicker is frequency-specific.
3) Injecting adenosine into the visual cortex can concentration-dependently increase the total sleep amount of mice

Adenosine was injected locally in the V1 cortex of mice to illustrate that the increase in the concentration of adenosine in the visual cortex could improve the sleep amount of mice. The administration cannulas were implanted in bilateral V1 brain regions of C57BU6J mice, and the mice were placed in the sleep recording cage after about 10 days of surgical recovery. After 3 days of adaptation, the artificial cerebrospinal fluid (ACSF), adenosine of 1.5 nmol/side or adenosine 4.5 nmol/side was microinjected into the V1 visual cortex at the beginning of the dark phase on days 1, 4 and 7 (the administration order was random, and the injection volume was 2µl/side), respectively, and the EEG/EMG of mice was recorded.

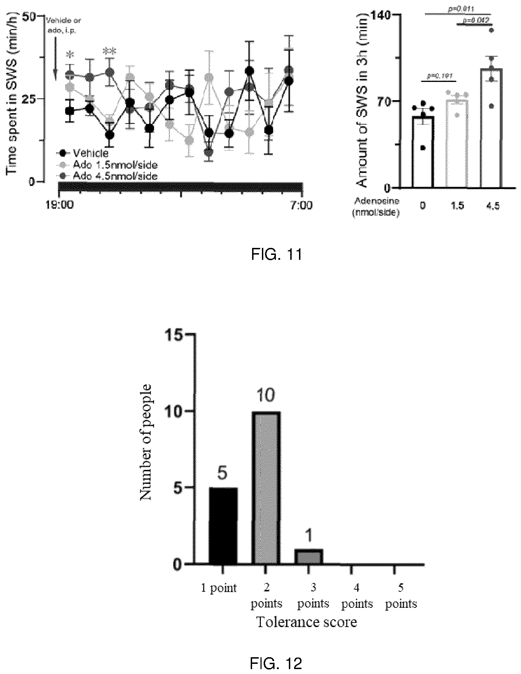

By comparing the sleep amount of mice after different treatments, as shown in FIG. 11, it is found that the amount of the non-rapid eye movement sleep of mice in the dose group of adenosine-injection with 4.5 nmol/side reached 3 hours in the dark phase, which significantly increases as compared to the control group; while the dose group of adenosine-injection with 1.5 nmol/side has a tendency to promote sleep but no significant difference as compared to the control group. It suggests that the sleep-promoting effect of adenosine locally administered in the visual cortex is dose- or concentration-dependent, and the experiment shows that the improvement of the total sleep amount of mice requires that the level of local adenosine in the visual cortex be increased to reach a certain threshold.

The above experiments support that the 40 Hz light flicker stimulus promotes sleep by enhancing the extracellular adenosine signal in the cortex, based on the frequency-specific effects of the adenosine production and sleep amount.

### Example 9. The 40 Hz light flicker stimulus is well tolerated and safe in healthy subjects

In order to advance the use of 40 Hz light flicker stimulus in clinical trials, we firstly recruited 15 healthy volunteers to test the tolerability and safety of the portable light flicker device of 40 Hz in the healthy subjects. In this experiment, the subjects without having breakfast was subjected to blood pressure and electrocardiogram measurement, blood sample collection, blood routine and liver and kidney function indicator tests at 8 a.m. on the previous morning. At 7:30 a.m. on the next morning, the light flicker from the portable light flicker device of 40 Hz was given for 30 minutes, then blood pressure and electrocardiogram were measured, and blood pressure samples were collected, blood routine and liver and kidney function indicators were tested, and questionnaire scale for adverse reactions was filled in. As shown in FIG. 12, compared with no light flicker, the 40 Hz light flicker stimulus does not significantly cause adverse reactions in the sensory system such as eyes and nose and in the neural center of the cerebrum, indicating that the 40 Hz light flicker stimulus and the portable device are well tolerated. They are also not shown significant abnormal changes in the relevant indicators in the blood pressure and blood tests. The above-mentioned results indicate that the 40 Hz light flicker and the portable device are well tolerated and safe in the healthy subjects.

**Table 1. Clinical data of healthy subjects (n=16)**

| Clinical indicator | Mean±standard deviation/frequency number (percentage) |
|---|---|
| Age (year) | 24.69±1.89 |
| Female (%) | 10 (62.5) |
| BMI (kg/m2) | 20.95±1.89 |
| Years of schooling (year) | 18±1.37 |
| Daily tea drinking (%) | 3 (18.75) |
| Daily coffee drinking (%) | 9 (56.25) |
| SF-36 score | 125.39±6.69 |
| PSQI score | 4.50±2.73 |
| Ess score | 6.94±3.21 |
| * Light flicker tolerance score | 1.75±0.58 |

| | |
|---|---|
| Note: data are presented as mean±standard deviation/mumber (percentage). * Light flicker tolerance score with full mark of 5 points, the lower the value, the better the tolerance. | |

Abbreviations: BMI: body mass index; SF-36: the MOS-36 item short form health survey; PSQI, Pittsburgh sleep quality index; Ess, Epworth Sleepiness Scale.

**Table 2. Safety assessment (blood and electrocardiogram test examination)**

| | | Before light flicker (n=16) | After light flicker (n=16) | ***p* value** |
|---|---|---|---|---|
| Heart rate (bpm), mean±SD Blood pressure (mmHg), mean±SD | | 71.13±9.86 | 69.94±11.86 | **0.534** |
| | Systolic pressure | 106.94±8.68 | 106.06±8.85 | **0.614** |
| | Diastolic pressure | 67.81±6.56 | 67.25±6.99 | **0.736** |
| Hyperlipidemia, n (%) | | 9(56.25) | 9(56.25) | **1** |
| Hyperuricemia, n (%) | | 4(25.00) | 5(31.25%) | **1** |
| Pathoglycemia, n (%) | | 0(0) | 0(0) | **1** |
| Hepatic insufficiency, n (%) | | 0(0) | 0(0) | **1** |
| Renal insufficiency, n (%) | | 0(0) | 0(0) | **1** |
| Anemia, n (%) | | 1(6.25) | 1(6.25) | **1** |
| Severe arrhythmia, n (%) | | 0(0) | 0(0) | **1** |
| Epileptic seizure, n (%) | | 0(0) | 0(0) | **1** |

| | | | | |
|---|---|---|---|---|
| Note: data are presented as mean ± standard deviation (SD)/frequency number (percentage). Paired t test or Chi-square test is used depending on the data type. | | | | |

**Table 3. Summary of adverse reaction events**

| Events | Number of occurrences | Severity | Relevance to the light flicker |
|---|---|---|---|
| Diarrhea | 1 | Mild | Not relevant |
| Eye dryness | 2 | Mild | Possibly relevant |
| Dizziness | 2 | Mild | Possibly relevant |
| Lacrimation | 1 | Mild | Possibly relevant |
| Sinus bradycardia | 2 | Mild | Not relevant |
| T-wave changes | 1 | Mild | Not relevant |
| ST segment elevation (early ventricular repolarization) | 1 | Mild | Not relevant |

### Example 10. The 40 Hz light flicker stimulus improves various sleep parameters in patients with chronic insomnia, including sleep latency, total sleep time, wakefulness after sleep onset, etc.

Objective of the study: This study aims to recruit patients with chronic insomnia and evaluate the efficacy of the 40 Hz light flicker stimulus on the patients with chronic insomnia.

Protocol of the study: In this study, patients meeting the diagnostic criteria of International Classification of Sleep Disorders (ICSD3) for chronic insomnia were recruited from the Second Affiliated Hospital of Wenzhou Medical University from February 2022 to December 2022. The polysomnography (PSG) was employed to record the patients' sleep. The study was an own-control design that the sleep baseline was recorded on the first night, and sleep was recorded again after 30 minutes of the intervention of the 40 Hz light flicker stimulus (3000 lux; duty ratio of 50%). The primary endpoints were: the significant decrease in sleep latency and the significant improvement in sleep efficiency. The secondary endpoints were: the significant extension in total sleep time, and the significantly reduction in the time and number of wakefulness after sleep onset.

Results of the study: A total of 27 patients were recruited in the study, aged 9.4±3.1 years, with a BMI of 15.40 (14.50-17.90) kg/m², including 14 females (51.85%).

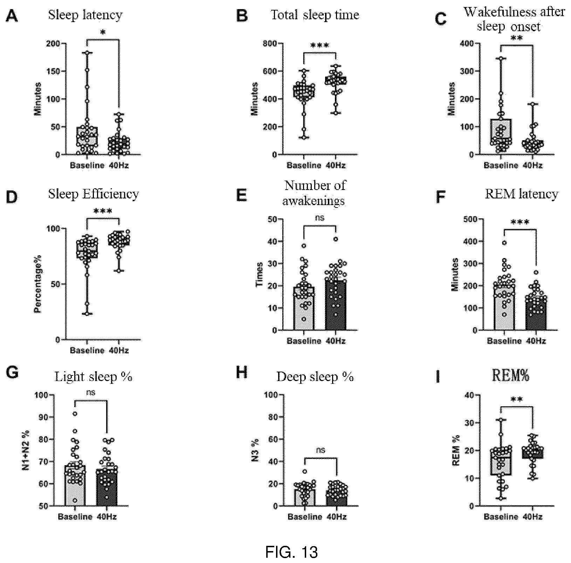

Findings of the study: The treatment of the 40 Hz light flicker stimulus significantly reduced the sleep latency (min) compared to the baseline (18.5 (10-29.5) vs. 32.50 (15-49.5), p =0.0233) (FIG. 13A). Meanwhile, the 40 Hz significantly extended the total sleep time (min) (528 (502-560) vs. 459 (411.5-497), p =0.0002) (FIG. 13B) and the wakefulness after sleep onset (min) (35.5 (28-51) vs. 57.5 (40-128.5), p =0.0012) (FIG. 13C), and also significantly improved the sleep efficiency (%) (89.3 (84.8-82.5) vs. 79.6 (73.2-87.5), p =0.0003) (FIG. 13D). There was a significant reduction in the sleep latency (min) of the rapid eye movement sleep (REM) (139.5 (107-182.5) vs. 199 (156.5-239.5), p =0.0004) (FIG. 13F), and an increase in the REM sleep proportion (%) (20.2 (17-21.3) vs. 17.4 (11-20.3), p =0.0038) (FIG. 13I). There were no significant effects on the number of number of awakenings, and the proportion of the light and deep sleep periods in the total sleep time (all p > 0.05) (FIG. 13E, G, H). Conclusion of the study: In this self-controlled clinical study of the patients with chronic insomnia, the 40 Hz light flicker stimulation for 30 minutes before bedtime significantly shortens the sleep latency, significantly extends the total sleep time, and significantly reduces time and number of wakefulness after sleep onset. Therefore, the 40 Hz light flicker stimulation for 30 minutes before bedtime can significantly improve the sleep initiation and the sleep maintenance in the patients with chronic insomnia.

### Example 11. Study on the main cellular origin for producing extracellular adenosine induced by the 40 Hz light flicker stimulus

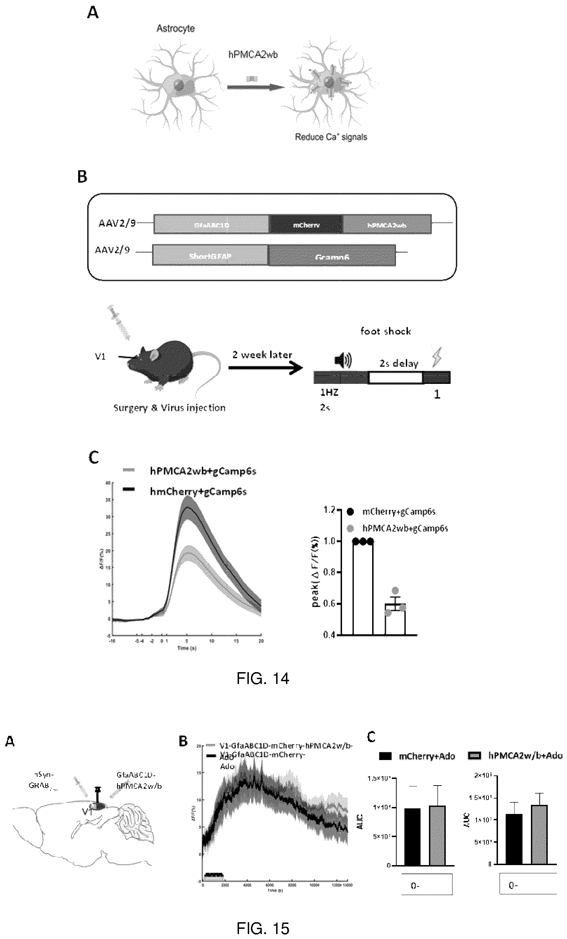

Under normal conditions, the production of extracellular adenosine may originate from astrocytes or neurons. Firstly, the virus-mediated knockdown of the astrocyte plasma membrane Ca²⁺ pump PMCA2 was used to confirmed whether astrocytes are the main cellular origin of the adenosine release promoted by the 40 Hz light flicker stimulus. hPMCA2w/b is a subtype of the plasma membrane Ca²⁺ pump PMCA, whose function is to constitutively extrude cytoplasmic Ca²⁺, and is capable of reducing the Ca²⁺ signal of astrocytes and reducing the activity (FIG. 14A). We infected a 1:1 mixture of AAV2/9-GfaABC1D-mCherry-hPMCA2w/b and AAV2/9-ShortGFAP-Gcamp6 into the V1 region of mice, and left the virus to express for 2 weeks. Then, the experimental method of foot shock (FIG. 14B) was employed to verify the feasibility of the virus-mediated knockdown of the plasma membrane CA²⁺ pump PMCA2 in the astrocyte. The experimental results showed that after the foot shock, the astrocyte reactivity was significantly reduced by 40% compared to the control side (AAV2/9-GfaABC1D-mCherry), indicating that the virus can indeed reduce the astrocyte activity (FIG. 14C).

To investigate if the astrocytes promote adenosine release in the 40 Hz light flicker, the mice injected with AAV2/9-GfaABC1D-mCherry-hPMCA2w/b virus were simultaneously injected with AAV2/9-GfaABC1D-mCherry-hPMCA2w/b and AAV2/9-hsyn-GRABAdo1.0 in the V1 region (1:1 mixing, FIG. 15A). After virus expression for 2 weeks, the 40 Hz light flicker stimulation was performed and the changes in the concentration of adenosine was recorded in real time. It was found that after the 40 Hz light flicker stimulation, the reduced increase and the change feature of the active adenosine concentration of astrocytes in the V1 region of mice were almost identical between the experimental group (mice with plasma membrane Ca²⁺ pump PMCA2 knockdown) and the control group (mice with normal plasma membrane Ca²⁺ pump PMCA2) (FIG. 15B-C), suggesting that the extracellular adenosine increased by the 40 Hz light flicker stimulus is not mainly originated from the astrocytes.

In order to illustrate that the neurons are the main cellular origins of the levels of extracellular adenosine induced by the 40 Hz light flicker stimulus, we measured the changes in the concentration of adenosine during the 40 Hz light flicker stimulation by the Caspase-3-specific damage to the neurons in the V1 region. The AAV2/9-hsyn-GRABAdo1.0 and rAAV-hSyn-taCasp3-T2A-TEVp were mixed at 1:1 and injected into the V1 region of mice. After virus expression for 2 weeks, the number of the neurons in the V1 region was significantly reduced observing by the fluorescence staining. Then the 40 Hz light flicker stimulation was performed and changes in the concentration of adenosine were recorded in real time. The results showed that extracellular adenosine significantly increased in the mice of the control group (with normal neurons in the V1 region) under the 40 Hz light flicker stimulation, while extracellular adenosine did not increase at all in the mice with missing neurons in the V1 region under the 40 Hz light flicker stimulus intervention (FIG. 16A-B). It is demonstrated that the neurons in the V1 region plays an important role in regulating the increase of the concentration of extracellular adenosine stimulated by the 40 Hz light flicker.

### Example 12. Glasses for regulating the adenosine release

As shown in FIG. 17, the main device components comprise: a micro-LED display screen, a shell, a flexible circuit board, an inner cover, left and right temples, etc. The device as a whole is in the shape of glasses, which is mainly to achieve two functions: (A) the LED lamp groups of different bands are fixedly clamped between the glasses shell and the inner cover, while the flexible circuit board is used to distribute around the display screen along the edge of the glasses frame, so that a variety of combinations of light flickering stimuli with different bands, frequencies, light intensities, time, etc., can be freely adjusted. (B) The micro-LED display screen is configured to cover the lens position of the glasses to display the optimal visual stimulus image based on the screening of the visual stimulus-adenosine concentration-sleep change. These two modes will be superior to the visual stimulus devices with fixed light flicker intensity and frequency in the market, greatly reducing the discomfort caused by the simple light flicker stimulus, and pioneering a new direction in the treatment of sleep disorder by the visual stimulus image.

## Claims

1. A device for regulating the release of adenosine in an organism, **characterized in that** it comprises a stimulus generator for generating a sound of 1-100 Hz and/or for generating a light flicker of 1-100 Hz and/or a full-screen visual stimulus.

2. The device according to claim 1, wherein the stimulus generator comprises visual stimulus generator and/or acoustic stimulus generator;
optionally, the visual generator emits a visual stimulus of 20-80 Hz; optionally, the frequency of the visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz;
optionally, the frequency of the visual stimulus comprises 30-60 Hz; optionally, the frequency of the visual stimulus comprises one or more of 30 Hz, 35 Hz, 40 Hz, 45 Hz, 50 Hz, 55 Hz, and 60 Hz;
optionally, the frequency of the visual stimulus comprises 35-45 Hz; optionally, the frequency of the visual stimulus comprises one or more of 35 Hz, 40 Hz, and 45 Hz; optionally, the frequency of the visual stimulus is 40 Hz;
optionally, the white light illuminance of the visual stimulus is 2000 lux or more; optionally, the wavelength of the monochromatic light of the visual stimulus is 480-530 nm;
optionally, the duty ratio of the visual stimulus is 20%-80%, preferably 30%-70%, or
40%-70%, or 45-55%;
optionally, the duty ratio of the visual stimulus is selected from one or more of 20%, 25%, 30%, 35%, 40%, 45%, 50%, 55%, 60%, 65%, 70%, 75%, and 80%;
optionally, the duty ratio of the visual stimulus is 50%.

3. The device according to claim 2, wherein the visual stimulus generator comprises a lamp and/or a display screen;
optionally, the lamp comprises CCFL lamp, LED lamp and/or OLED lamp;
optionally, the display screen comprises CCFL display screen, LED display screen, and/or OLED display screen.

4. The device according to claim 3, wherein the lamp emits white light and/or monochromatic light;
optionally, the monochromatic light comprises one or more of ultraviolet light, blue light, green light, red light and near-infrared ray, preferably blue light and/or green light;
optionally, the center wavelength of the ultraviolet light is 315-400 nm, 320-395 nm, or 325-390 nm, or 330-385 nm; preferably 380 nm;
optionally, the center wavelength of the blue light is 450-490 nm, or 455-488 nm, or
460-485 nm; preferably 480 nm;
optionally, the center wavelength of the green light is 500 nm~560 nm, or 505 nm555 nm, or 510 nm~550 nm, or 515 nm~545 nm, or 520 nm~540 nm, or 525 nm~545 nm, or 530 nm~535 nm; preferably 530 nm;
optionally, the center wavelength of the red light is 600-680 nm, or 595-675 nm, or 590-670 nm, or 585-665 nm, or 580-650 nm; preferably 650 nm;
optionally, the center wavelength of the near-infrared ray is 780-1100 nm, or 790-1090 nm, or 800-1080 nm, or 810-1070 nm, or 820-1060 nm, or 830-1050 nm, or 840-1040 nm, or 850-1030 nm; preferably 850 nm;
optionally, the white light illuminance is 2-6000 Lux, and the monochromatic light illuminance is 2-500 Lux;
optionally, the white light illuminance emitted by the lamp is 500-3000 Lux, optionally, the white light illuminance emitted by the lamp comprises one or more of 500 Lux, 1000 Lux, 2000 Lux and 3000 Lux.

5. The device according to claim 3 or 4, wherein the display screen displays a grating image; optionally, the contrast ratio of the grating image is 10%-100%;
optionally, the grating image comprises one or more of moving grating image, flickering grating image, flickering raster image and flipping grating image; optionally, the contrast ratio of the moving grating is 10%-100%; optionally, the orientation of the moving grating is 0-180°; optionally, the spatial frequency of the moving grating image is 0.05-30 cycle/deg; e.g., 0.05-10 cycle/deg; optionally, the moving speed of the moving grating image is 1-10 Hz;
optionally, the contrast ratio of the flipping grating is 10%-100%; optionally, the flipping frequency of the flipping grating is 20-60 Hz or 30-50 Hz, preferably 40 Hz; optionally, the spatial frequency of the flipping grating is 0.1-30 cpd, or 0.1 cpd; optionally, the contrast ratio of the flipping grating is 10%-100%, 70-90%, preferably 80%; optionally, the average brightness of the flipping grating is 100-500 cd/m², or 140-180 cd/m², or 150-170 cd/m², or 160 cd/m²;
optionally, the contrast ratio of the flickering raster image is 10%-100%; optionally, the frequency of the flickering raster image is 1-100 Hz; optionally, the raster size of the flickering raster image is 0.5-10 deg.

6. The device according to any one of claims 1-5, wherein the device comprises mobile phone, tablet, or portable wearable device.

7. The device according to claim 6, wherein the portable wearable device is selected from glasses, blinder, mask, hat, headband, helmet, bracelet or watch; optionally, the glasses comprise VR glasses.

8. The device according to claim 7, wherein the portable wearable device comprises glasses frame; optionally, the portable wearable device also comprises glasses temples or hair band;
optionally, a lamp is provided at the edge of the lens enclosed by the glasses frame; optionally, the lamp comprises CCFL lamp, LED lamp and/or OLED lamp; optionally, a display screen is provided at the center of the lens enclosed by the glasses frame; optionally, the display screen comprises CCFL display screen, LED display screen, and/or OLED display screen.

9. The device according to claim 8, wherein the lamp comprises monochromatic light lamp and white light lamp;
optionally, the illuminance of the monochromatic light lamp is 2-500 lux; optionally, the illuminance of the white light is 2-6000 lux;
optionally, the lamp comprises blue light lamp, green light lamp, red light lamp and white light lamp.

10. The device according to claim 8 or 9, wherein the display screen displays a grating image which comprises vertical moving grating, horizontal moving grating and/or flickering raster;
optionally, the vertical moving grating is parameterized to be oriented at 0°; optionally, the spatial frequency of the vertical moving grating is 0.2 cpd; optionally, the time frequency of the vertical moving grating is 1 Hz; optionally, the contrast ratio of the vertical moving grating is 50%;
optionally, the horizontal moving grating is parameterized to be oriented at 90°; optionally, the spatial frequency of the horizontal moving grating is 0.2 cpd; optionally, the time frequency of the horizontal moving grating is 1 Hz; optionally, the contrast ratio of the horizontal motion grating is 50%.

11. Use of the device according to any one of claims 1-10, comprising the use for promoting the release of adenosine in an organism;
optionally, the use comprises increasing the concentration of adenosine within an organism;
optionally, the use for increasing the concentration of extracellular adenosine of neurons.

12. The use according to claim 11, comprising the treatment of neurological disease, cardiovascular disease and/or ophthalmic disease;
optionally, the neurological disease comprises Parkinson's disease, depression, anxiety, dementia, Alzheimer's disease, post-traumatic stress disorder, hyperactivity disorder, childhood autism and/or attention deficit disorder;
optionally, the cardiovascular disease comprises coronary heart disease and/or arrhythmia;
optionally, the ophthalmic disease comprises myopia, strabismic amblyopia, and senile retinopathy.

13. The use according to claim 11, comprising the improvement of sleep disorder, optionally, improving the sleep disorder by a visual stimulus of 20-80 Hz, improving the sleep disorder by a visual stimulus of 30-60 Hz, optionally, improving the sleep disorder by a visual stimulus of 35-45 Hz, optionally, improving the sleep disorder by a visual stimulus of 40 Hz.

14. A method for regulating the release of adenosine in an organism, comprising the following steps:
delivering a stimulus of 1-100 Hz to a subject by using the device according to any one of claims 1-10, the delivery is a non-invasive delivery; optionally, the stimulus comprises visual stimulus and/or acoustic stimulus.

15. The method according to claim 14, wherein the frequency of the visual stimulus is 1-100 Hz, optionally, the frequency of the visual stimulus is 20-80 Hz; the frequency of the visual stimulus comprises one or more of 20 Hz, 25 Hz, 30 Hz, 35 Hz, 40 Hz, 45 Hz, 50 Hz, 55 Hz, 60 Hz, 65 Hz, 70 Hz, 75 Hz and 80 Hz;
optionally, the frequency of the visual stimulus comprises 30-60 Hz; optionally, the frequency of the visual stimulus comprises one or more of 30 Hz, 35 Hz, 40 Hz, 45 Hz, 50 Hz, 55 Hz, and 60 Hz;
optionally, the frequency of the visual stimulus comprises 35-45 Hz; optionally, the frequency of the visual stimulus comprises one or more of 35 Hz, 40 Hz, and 45 Hz; optionally, the frequency of the visual stimulus is 40 Hz;
optionally, the white light illuminance of the visual stimulus is 2000 lux or more; optionally, the wavelength of the monochromatic light of the visual stimulus is 480-530 nm;
optionally, the duty ratio of the visual stimulus is 20%-80%, preferably 30%-70%, or 40%-70%, or 45-55%;
optionally, the duty ratio of the visual stimulus is selected from one or more of 20%, 25%, 30%, 35%, 40%, 45%, 50%, 55%, 60%, 65%, 70%, 75%, and 80%; optionally, the duty ratio of the visual stimulus is 50%.

16. The method according to claim 15, wherein the visual stimulus comprises light flicker stimulus and/or image stimulus;
optionally, the light flicker comprises white light and/or monochromatic light;
optionally, the image comprises grating image.

17. The method according to claim 16, wherein the monochromatic light comprises one or more of ultraviolet light, blue light, green light, red light and near-infrared ray, preferably blue light and/or green light;
optionally, the center wavelength of the ultraviolet light is 315-400 nm; the center wavelength of the blue light is 450-490 nm; the center wavelength of the green light is 500 nm~560 nm; the center wavelength of the red light is 600-680 nm; the center wavelength of the near-infrared ray is 780-1100 nm;
optionally, the center wavelength of the ultraviolet light is 320-395 nm, or 325-390 nm, or 330-385 nm;
optionally, the center wavelength of the blue light is 455-488 nm, or 460-485 nm; optionally, the center wavelength of the green light is 505 nm~555 nm, or 510 nm~550 nm, or 515 nm~545 nm, or 520 nm~540 nm, or 525 nm~545 nm, or 530 nm~535 nm;
optionally, the center wavelength of the red light is 595-675 nm, or 590-670 nm, or 585- 665 nm, or 580-650 nm;
optionally, the center wavelength of the near-infrared ray is 790-1090 nm, or 800-1080 nm, or 810-1070 nm, or 820-1060 nm, or 830-1050 nm, or 840-1040 nm, or 850-1030 nm; optionally, the center wavelength of the ultraviolet light is 380 nm; the center wavelength of the blue light is 480 nm; the center wavelength of the green light is 530 nm; the center wavelength of the red light is 650 nm; the center wavelength of the near-infrared ray is 850 nm;
optionally, the white light illuminance is 2-6000 Lux, and the monochromatic light illuminance is 2-500 Lux;
optionally, the white light illuminance emitted by the lamp is 500-3000 Lux, optionally, the white light illuminance emitted by the lamp comprises one or more of 500 Lux, 1000 Lux, 2000 Lux and 3000 Lux.

18. The method according to claim 16 or 17, wherein the contrast ratio of the grating image is 10%-100%;
optionally, the grating image comprises one or more of moving grating image, flickering grating image, flickering raster image and flipping grating image;
optionally, the contrast ratio of the moving grating is 10%-100%; optionally, the orientation of the moving grating is 0-180°; optionally, the spatial frequency of the moving grating image is 0.05-30 cycle/degree; optionally, the moving speed of the moving grating image is 1-10 Hz;
optionally, the contrast ratio of the flipping grating is 10%-100%; optionally, the flipping frequency of the flipping grating is 20-60 Hz or 30-50 Hz, preferably 40 Hz; optionally, the spatial frequency of the flipping grating is 0.1-30 cpd, or 0.1 cpd; optionally, the contrast ratio of the flipping grating is 10%-100%, 70-90%, preferably 80%; optionally, the average brightness of the flipping grating is 100-500 cd/m², or 140-180 cd/m², or 150-170 cd/m², or 160 cd/m²;
optionally, the contrast ratio of the flickering raster image is 10%-100%; optionally, the frequency of the flickering raster image is 1-100 Hz; optionally, the raster size of the flickering raster image is 0.5-10 degree.

19. An assessment method for the efficacy of a drug or a medical device, comprising analyzing the change of the concentration of adenosine within an organism.

20. The assessment method according to claim 19, the drug or medical device is used for the treatment of neurological disease, the treatment of cardiovascular disease and/or the improvement of sleep disorder;
optionally, the neurological disease comprises Parkinson's disease, depression, anxiety, dementia, Alzheimer's disease, post-traumatic stress disorder, hyperactivity disorder, childhood autism and/or attention deficit disorder;
optionally, the cardiovascular disease comprises coronary heart disease and/or arrhythmia.
